# FASCICULE DE BREVET EUROPEEN

(11) **EP 1 062 358 B1**
(45) Date de publication et mention de la délivrance du brevet: **04.06.2003**
(21) Numéro de dépôt: 99909036.8
(22) Date de dépôt: 18.03.1999
(51) Int. Cl.: C12P 17/12, C07F 9/38, C07D 211/70

(54) **NOUVEAU PROCEDE DE PREPARATION DE LA FEXOFENADINE**
VERFAHREN ZUR HERSTELLUNG VON FEXOFENADIN
NOVEL METHOD FOR PREPARING FEXOFENADINE

(30) Priorité: 19.03.1998 FR 9803349
(43) Date de publication de la demande: 27.12.2000
(73) Titulaire: Aventis Pharma S.A., 92160 Antony (FR)
(72) Inventeur: AZERAD, Robert, F-91130 Ris Orangis (FR); BITON, Jacques, F-60610 La Croix Saint Ouen (FR); LACROIX, Isabelle, F-94600 Choisy le Roi (FR)
(86) Numéro de dépôt international: FR9900625
(87) Numéro de publication internationale: WO99047693

(56) Documents cités:
- EP-A- 0 864 653
- WO-A-95/00480
- CHAN K Y ET AL: "DIRECT ENANTIOMERIC SEPARATION OF TERFENADINE AND ITS MAJOR ACID METABOLITE BY HIGH-PERFORMANCE LIQUID CHROMATOGRAPHY, AND THE LACK OF STEREOSELECTIVE TERFENADINE ENANTIOMER BIOTRANSFORMATION IN MAN" JOURNAL OF CHROMATOGRAPHY, BIOMEDICAL APPLICATIONS, vol. 571, no. 1/02, 15 novembre 1991, pages 291-297, XP002046562

## Description

La présente invention a pour objet un nouveau procédé de préparation de la Fexofénadine.

La Fexofénadine est un médicament qui possède une activité antihistaminique significative et qui est dépourvu d'effets secondaires. (Efficacy and safety of Fexofenadine hydrochloride for treatment of seasonal allergic rhinitis, Bernstein D.I. et al, Ann. Allergy-Asthma-Immunol. (1997) 79(5) 443-448.

Ce composé est le principal métabolite de la Terfenadine, lui-même agent antihistaminique (D. Mc Tavish, KL Goa et M. Ferill, "terfenadine : an updated review of its pharmacological properties and therapeutic efficiency, Drug 39, 552-574 (1989)).

La Fexofénadine est actuellement préparée par voie chimique, en de nombreuses étapes avec un rendement inférieur à 10 % (Brevets US 5.578.610 et 5.581.011).

D'autres procédés de préparation sont également décrits : Dans Journal of Chromatography, Biomédical application, vol. 571, n° ½, 15/11/91, pages 291-297, Chan K Y et al. décrivent une méthode de séparation des énantiomères de la Terfénadine par chromatographie ainsi que la détermination et la composition énantiomérique de 1a Féxofénadine issue de 1a métabolisation in vivo de 1a Terfénadine.

La demande WO-A-95 00480 décrit des procédés de préparation de 1a Féxofénadine par voie chimique.

La demande EP-A-864653 décrit un procédé de transformation microbiologique de la Terfénadine en Féxofénadine en utilisant comme souche soit *Cunninghamella* soit *Absidia.*

La demanderesse se propose donc de trouver une autre voie de synthèse de la Fexofénadine. Le choix s'est alors porté sur une méthode de bioconversion en utilisant l'un des deux types de microorganismes très spécifiques suivants :
soit le champignon filamenteux de genre *Absidia Corymbifera* et notamment *Absidia Corymbifera* CP 63-1800,
soit un Streptomyces et notamment le *Streptomyces platensis* NRRL 2364.
La spécificité de la bioconversion avec ces microorganismes était inattendue : parmi de nombreuses souches étudiées lors d'un screening, ce sont les seules qui permettent l'obtention de la Fexofénadine avec un bon rendement et peu ou pas de produits secondaires.

L'invention a pour objet un procédé de préparation des composés de formule (I) : 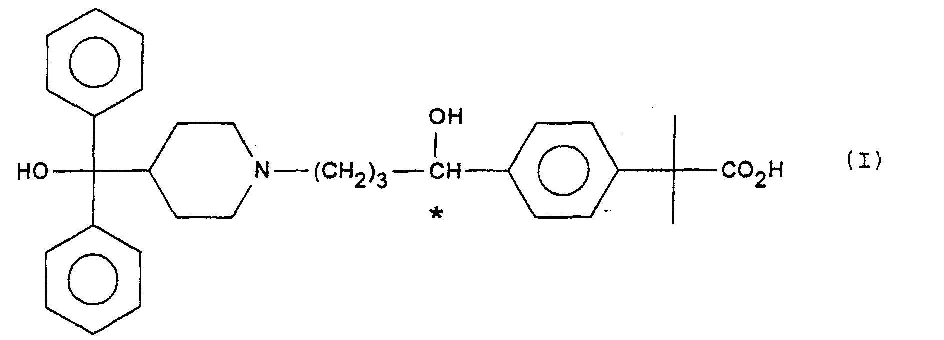 caractérisé en ce qu'on effectue une bioconversion du ou des composés de formule (II) : 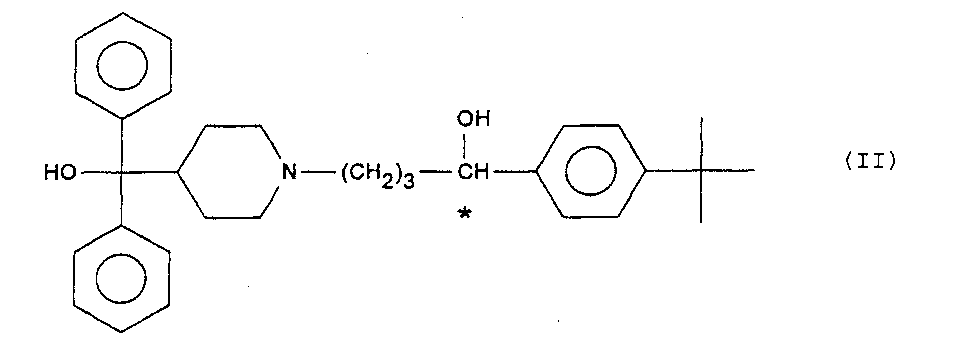 avec soit une culture d'un microorganisme de genre *Absidia corymbifera,* soit une culture d'un microorganisme de genre *Streptomyces, à* un pH compris entre 5,0 et 8,0, afin d'obtenir le ou les composés de formule (I) attendus, qui le cas échéant sont isolés, purifiés et/ou salifiés, les composés de formules (I) ou (II) pouvant être sous les deux formes énantiomères possibles, isolées ou en mélanges. L'astérisque indique la position du carbone asymétrique.

L'invention a particulièrement pour objet un procédé de préparation tel que décrit ci-dessus dans lequel l'*Absidia* c*orymbifera* est *Absidia corymbifera* LCP 63-1800 ou dans lequel le *Streptomyces* est *Streptomyces platensis* NRRL 2364.

La Fexofénadine est un mélange racémique des énantiomères de formule (I). Le procédé a donc plus particulièrement pour objet le procédé tel que décrit précédemment dans lequel on effectue une bioconversion de la Terfenadine, correspondant à un mélange racémique des deux énantiomères de formule (II), afin d'obtenir la Fexofénadine, correspondant à un mélange racémique des énantiomères de formule (I).

Les *Absidia corymbifera* et notamment *Absidia corymbifera* LCP 63-1800 sont accessibles au Laboratoire de Cryptogamie du Muséum d'Histoire Naturelle de Paris.

Parmi les *Streptomyces* susceptibles d'être utilisés dans le procédé, objet de cette demande, on peut citer les *Streptomyces* suivants :
*· Streptomyces albus*
*· Streptomyces ambofaciens* ATCC 15154
*· Streptomyces antibioticus* ATCC 31771
*· Streptomyces aureofaciens* ATCC 10762
*· Streptomyces djakartensis*
*· Streptomyces erythraeus*
*· Streptomyces felleus* DSM 40130
*· Streptomyces fradiae* W3554
*· Streptomyces griseus* NRRL B150
*· Streptomyces* JSP-2 (FH2126)
*· Streptomyces lividans* JT46/pCS2
*· Streptomyces narbonensis* FH 2102
*· Streptomyces olivaceus* ATCC 3335
*· Streptomyces platensis* ATCC 13865
*· Streptomyces platensis* NRRL 2364
*· Streptomyces rimosus* 2234
*· Streptomyces venezuelae* NRRL B-2446.

Il est connu de l'homme du métier que l'extrême simplicité des cellules bactériennes, sans noyau distinct, permet de les classer comme procaryotes. C'est le cas des *Streptomyces,* bactéries filamenteuses, qui sont aérobies et gram-positif. A l'opposé, les autres microorganismes sont nommés eucaryotes comme, par exemple, les champignons filamenteux (fungi) et tout particulièrement *Absidia corymbifera.* Leurs cellules se différencient notablement des procaryotes par la présence d'un noyau et de nombreux organites cytoplasmiques.

Le procédé ci-dessus décrit, objet de la présente demande, présente de nombreux avantages. D'une part, il évite la voie chimique qui nécessite de nombreuses étapes de synthèses accompagnées des procédés d'isolement nécessaires à chacune de ces étapes réactionnelles. Dans le cas d'une industrialisation, cette voie peut s'avérer coûteuse et polluante.

Dans le cas du procédé objet de cette demande, une seule opération est nécessaire et l'étape de purification éventuelle n'a essentiellement pour objet que d'éliminer un produit secondaire qui peut se former lors de la bioconversion, à savoir le triolphosphate qui correspond à l'alcool non encore oxydé en acide, estérifié sous la forme d'un phosphate (formule (IIIb)) décrit plus bas.

D'autre part ce procédé se prête bien à l'industrialisation. En opérant avec une concentration de produit de départ de 0,5 g/l, le rendement dépasse les 70 % par rapport au produit de départ.

Parmi les autres avantages de l'utilisation de la voie microbiologique par rapport à la voie chimique, on peut souligner l'aspect non polluant de cette technique, toutes les opérations s'effectuant en milieux aqueux.

La purification s'effectue selon les méthodes connues de l'homme du métier. Il peut s'agir d'une purification par cristallisation, par chromatographie ou par résine échangeuse d'ions.

Les réactions de salification peuvent être effectuées dans des conditions usuelles. On opère par exemple en présence de soude éthanolique. On peut également utiliser un sel de sodium tel que le carbonate ou le carbonate acide de sodium ou de potassium. Les sels obtenus peuvent être des sels de métaux alcalins ou alcalinoterreux ou d'ammonium éventuellement substitué.

La mise en oeuvre de l'oxydation s'effectue selon les méthodes qu'on applique couramment pour l'oxydation microbiologique des molécules organiques à l'aide de cultures de champignons filamenteux (Holland HL, Organic synthesis with oxidative enzymes. VCH publisher, Inc, New York 1992 ; Lacroix I, Biton J and Azerad R, Microbial biotransformation of a synthetic immunomodulating agent HR325, Bioorg. Med. Chem. (1997) 7, 1369-1380 ; Sebek OK, Fungal transformations as a useful method for the organic synthesis of organic compounds, Mycologia 75(2) 383-394, 1983 Azerad, R. Microbial models for drug metabolism, Advances in Biochemical Engineering and Biotechnology, Vol. 63, page 169, 1999.

Ainsi, on détermine tout d'abord par voie analytique, en particulier par chromatographie en couche mince ou HPLC, dans des essais préalables, les conditions de fermentation les plus favorables, comme par exemple le choix du milieu nutritif, du solvant de substrat approprié, de la concentration de substrat, des conditions techniques telles que la température, aération, pH, et des périodes optimum pour la culture, l'addition de substrat et le contact du substrat avec le microorganisme.

Dans une première phase, on effectue la culture à partir d'un inoculum (spores ou mycélium) d'*Absidia corymbifera* LCP 63-1800 ou d'une bactérie du genre *Streptomyces,* en particulier *Streptomyces platensis* NRRL 2364, dans un milieu nutritif liquide à un pH initial de 5 à 7 et à une température de 20 à 30°C, de préférence de 26 à 28°C. L'aération est assurée par agitation rotative des récipients de culture (150 à 250 rpm) ou, en fermenteur, par introduction d'air à un débit d'environ 1 l/min et par litre de bouillon de culture.

Après un temps variant de 24 à 72 heures, de préférence 60 à 65 heures, on ajoute la terfénadine à une concentration de 0,1 à 1 g/litre, de préférence 0,4 à 0,6 g/litre, en solution dans un solvant organique miscible à l'eau, tel que l'éthanol, l'acétone, le tétrahydrofuranne, la diméthylformamide ou le diméthylsulfoxyde (5 à 20 ml/litre de culture), de préférence l'éthanol (10 ml/litre). L'incubation est poursuivie dans les mêmes conditions que la culture. Le pH est éventuellement réajusté et maintenu à une valeur de 5,0 à 8,0. La transformation du substrat est avantageusement suivie par analyse par HPLC du surnageant d'incubation ou chromatographie en couche mince d'échantillons extraits par un solvant organique. En général après 48 à 200 heures, il s'est formé des quantités suffisantes de Fexofénadine.

Une autre procédure consiste à séparer la biomasse du milieu de culture par filtration, la laver à l'eau ou une solution tamponnée de pH neutre, puis la remettre en suspension dans un tampon adéquat, par exemple un tampon phosphate 0,05 M à pH 7, puis ajouter le substrat et poursuivre l'incubation comme décrit précédemment.

L'isolement et la purification des produits du procédé s'effectuent d'une manière connue en soi. Par exemple, on peut extraire les produits du procédé avec un solvant organique, de préférence l'acétate d'éthyle, ou par adsorption sur une colonne hydrophobe, suivie d'élution par un solvant organique, évaporer le solvant organique, séparer et purifier les produits par chromatographie sur colonne ou par cristallisation.

L'invention a donc plus particulièrement pour objet, le procédé tel que décrit précédemment dans lequel les conditions de biotransformation sont les suivantes :
concentration en terfenadine ajoutée de 0,5 g/l à 10 g/l, et de préférence DE 0,5 g/l à 5 g:l, pH évoluant entre 5,0 et 8,0, température comprise entre 26°C et 28°C et aération par introduction d'un débit d'air d'environ 1 l/mn et par litre de bouillon de culture.

Un des objectifs de l'invention est d'ajuster le pH au cours de l'incubation et éventuellement le maintenir à une valeur optimisée, en vue de minimiser la formation de produits secondaires.

La transformation de la Terfénadine (II) en Fexofénadine (I) par *A. corymbifera* ou *S. platensis* met en jeu plusieurs étapes successives, avec la formation intermédiaire du triol (IIIc) et celle de deux produits secondaires non souhaités, la Terfénadine-phosphate (IIIa) et le triolphosphate (IIIb) selon le schéma ci-dessous :

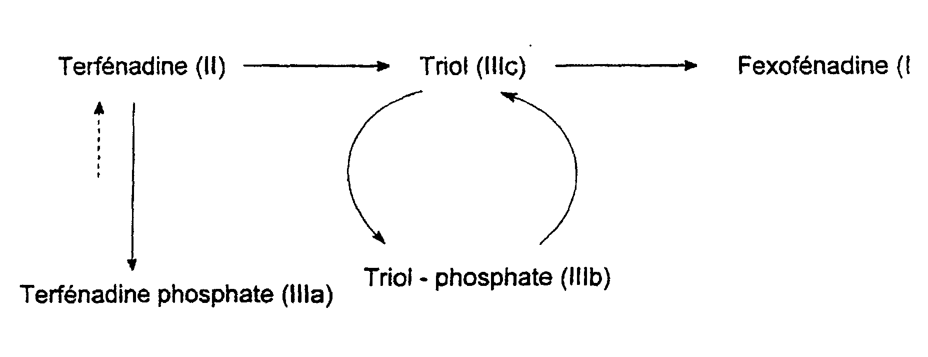

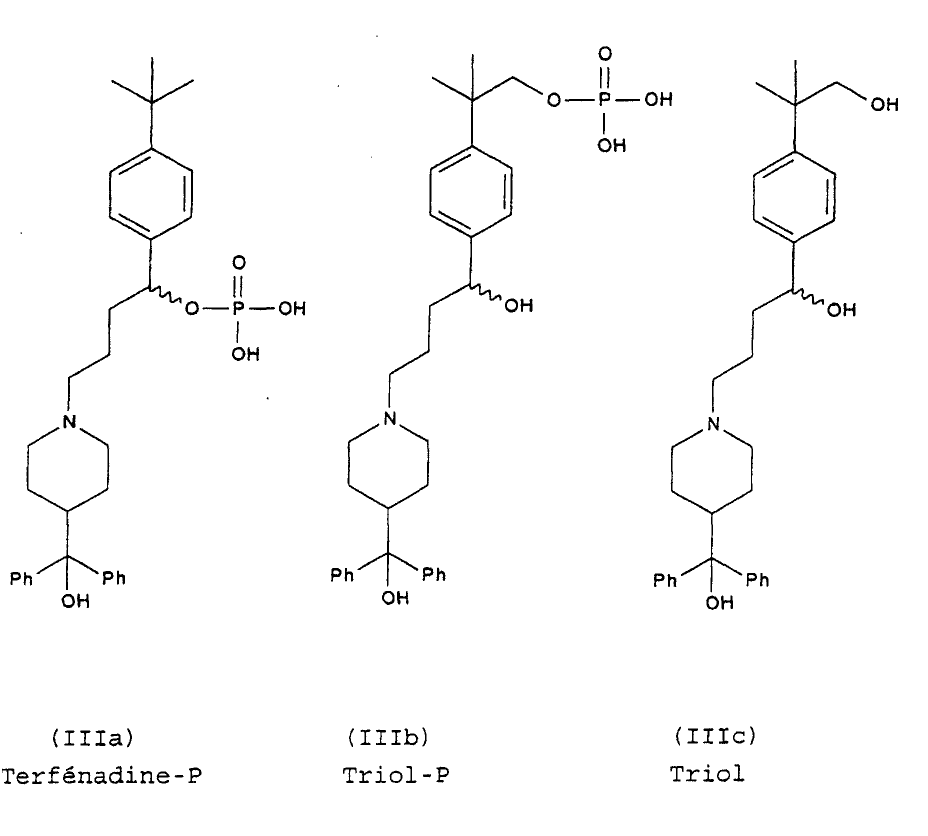

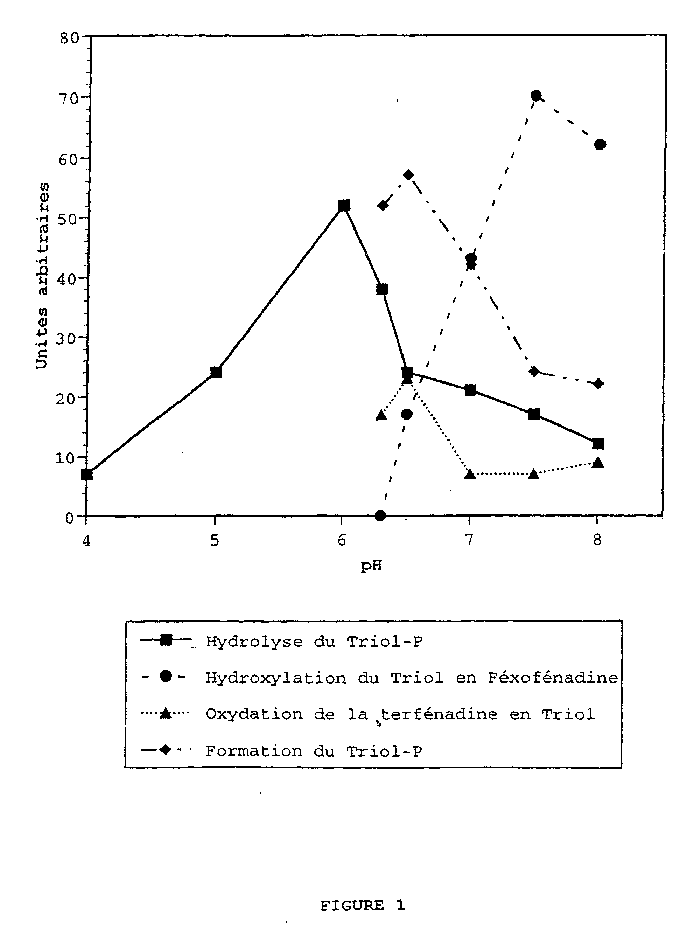

L'oxydation initiale de la terfénadine (I) en triol (IIIc), produit par le micro-organisme dans le milieu d'incubation, est favorisée par un pH légèrement acide ou neutre(6 < pH < 7) (tableau 2). La formation (apparemment irréversible) de Terfénadine-phosphate (IIIa) reste très limitée dans toutes les conditions étudiées et inférieure à 1-2 %. Par contre, la formation de triol-phosphate (IIIb) représente une part importante des produits d'oxydation accumulés dans le milieu, particulièrement lorsque l'incubation est menée à pH neutre ou alcalin (pH 7,0-8,0) comme le montre le tableau 3 (voir aussi figure 1) ; c'est le pH vers lequel évolue naturellement le milieu d'incubation. Cependant, à pH légèrement plus acide (pH optimum ∼6), une phosphatase d'origine fongique, présente dans le milieu d'incubation, catalyse l'hydrolyse rapide du triol-phosphate (IIIb) (tableau 4, figure 1), tandis que l'oxydation irréversible du triol en fexofénadine est favorisée à pH plus élevé (pH ≥ 7) (tableau 5, figure 1).

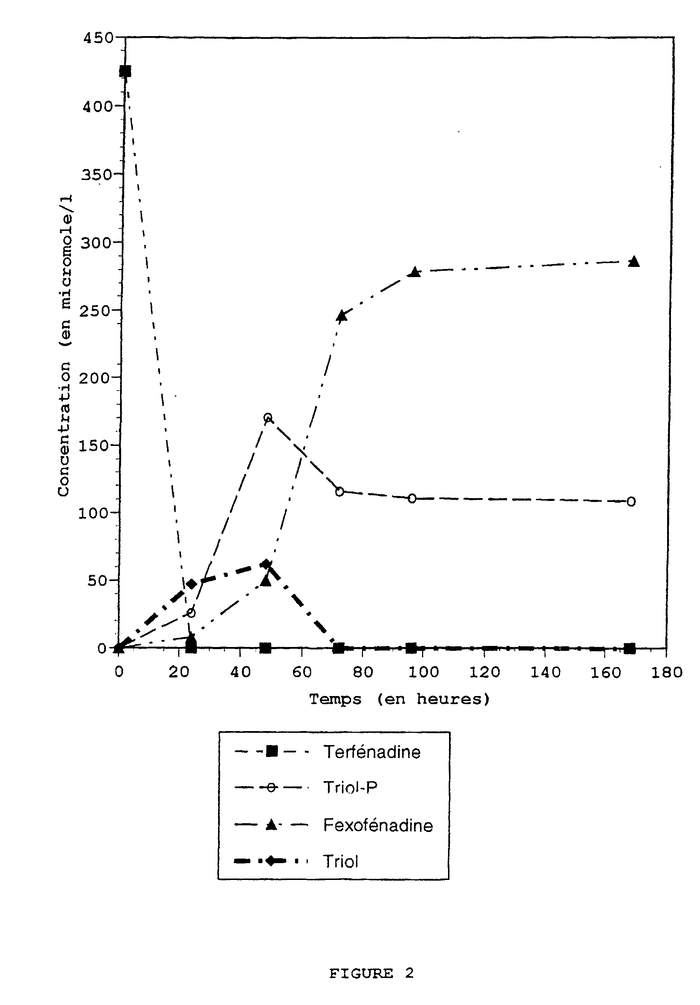

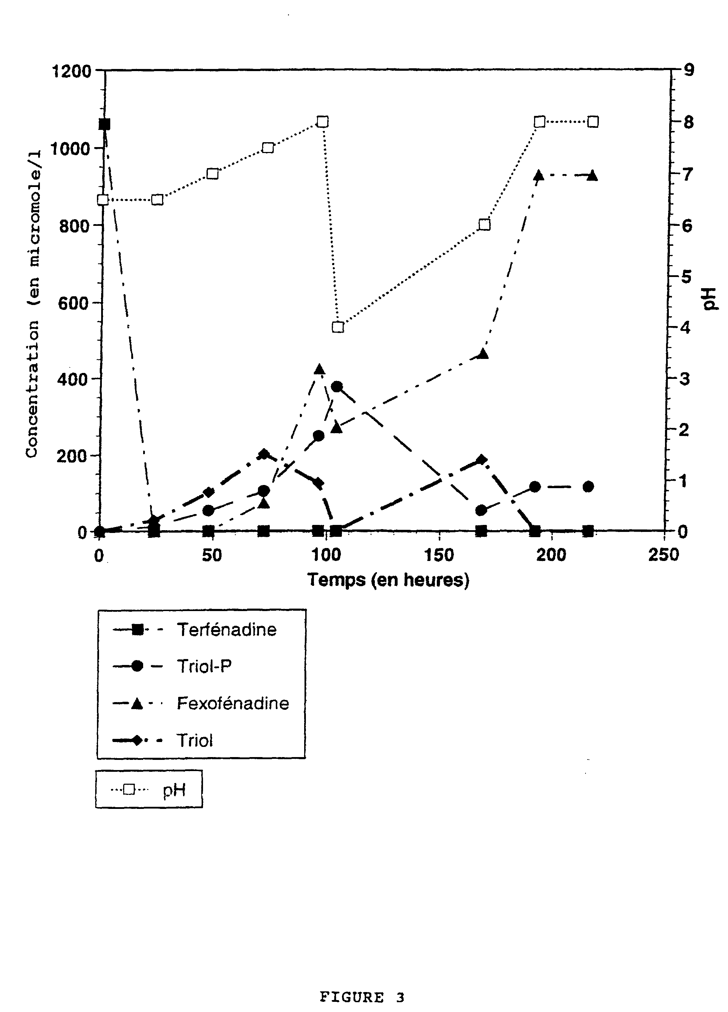

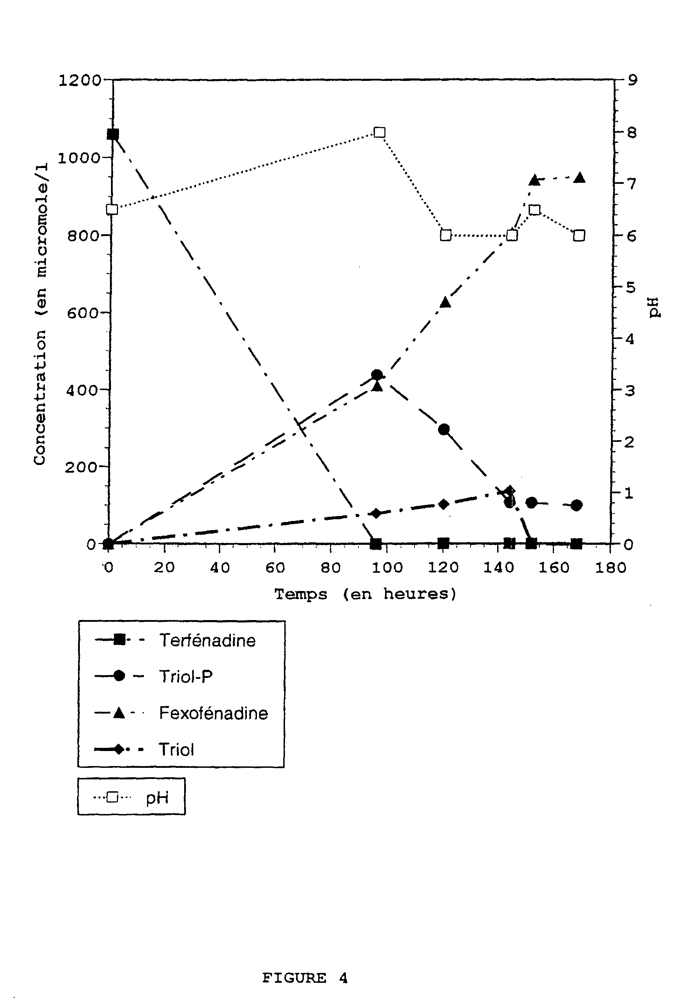

II en résulte plusieurs stratégies possibles pour transformer la quasi-totalité de la Terfénadine en Fexofénadine (si on ne tient pas compte de la très faible quantité de Terfénadine-phosphate formée) :
- procéder d'abord à l'incubation dans le milieu de culture initialement à un pH d'environ 6,5, sans contrôle du pH, ce qui conduit (en même temps que le pH évolue vers 8,0-8,5) à une transformation complète de la terfénadine en un mélange stationnaire stable de triol-phosphate (IIIb) et de fexofénadine (Fig. 2) (exemples 2 et 3), puis abaisser le pH à une valeur comprise entre 3,5 et 4 afin de favoriser la transformation de (IIIb) en (IIIc) et le laisser remonter lentement et spontanément vers 6,0, jusqu'à disparition complète du triol-phosphate (IIIb), puis réajuster et maintenir le pH aux environs de 8,0, jusqu'à transformation du triol (IIIc) en fexofénadine (Fig. 3) (exemple 4).
- procéder de la même manière à une incubation dans le milieu de culture, initialement à un pH d'environ 6,5, sans contrôle du pH, (le pH évolue naturellement vers 8,0-8,5) puis abaisser et maintenir celui-ci à une valeur comprise entre 6,0 et 6,5, pH auquel le triol-phosphate (IIIb) est assez rapidement hydrolysé, comme résultat de l'équilibre des activités phosphatase-phosphorylase à ce pH, tandis que le triol (IIIc) est lui-même assez rapidement oxydé, jusqu'à transformation presque complète en fexofénadine (fig. 4) (exemple 5).

L'invention a donc plus particulièrement pour objet :
- un procédé tel que défini plus haut dans lequel le pH initialement à environ 6,5,
   · évolue naturellement à 8,0-8,5, ce qui conduit à un mélange de triol-phosphate (IIIb) et de composé de formule (I),
   · est ensuite abaissé à une valeur comprise entre 3,5 et 4 afin de favoriser la transformation du composé intermédiaire de formule (IIIb) en composé intermédiaire de formule (IIIc),
   · puis évolue naturellement à environ 6,0 jusqu'à disparition complète du composé (IIIb),
   · et enfin est réajusté et maintenu à environ 8,0 jusqu'à transformation du composé (IIIc) en Fexofénadine.
- un procédé tel que défini plus haut dans lequel le pH initialement à environ 6,5
   . évolue naturellement à 8,0-8,5,
   . puis est abaissé et maintenu à une valeur comprise entre 6,3 et 6,8.
- un procédé tel que défini plus haut dans lequel au cours de l'étape de purification, l'extraction du produit de formule (I) est effectuée dans l'acétate d'éthyle.

Enfin, l'invention a également pour objet à titre de composé intermédiaire, le composé de formule (IIIc), telle que définie plus haut.

Les exemples suivants illustrent l'invention sans toutefois la limiter.

### Préparation des différents milieux de culture

Milieu A : Corn steep liqueur, 10 ml ; NaNO₃, 2 g ; MgSO₄, 7H₂O, 0,5 g ; FeSO₄,7H₂O, 0,02 g ; KCl, 0,5 g pour 900 ml d'eau distillée. Tampon phosphate (K₂HPO₄, 2 g ; KH₂PO₄, 1 g dans 40 ml d'eau distillée) et glucose, 30 g dans 60 ml d'eau distillée, ajoutés au moment de l'ensemencement.
Milieu B : Peptone de soja, 5 g ; extrait de levure, 5 g ; MgSO₄7H₂O, 0,5 g ; NaNO₃, 2 g ; KCl, 0,5 g ; FeSO₄, 7H₂O, 0,02 g pour 900 ml d'eau distillée. Tampon phosphate (K₂HPO₄, 2 g ; KH₂PO₄, 1 g dans 40 ml d'eau distillée) et glucose, 30 g dans 60 ml d'eau distillée, ajoutés au moment de l 'ensemencement.
Milieu C : Peptone de soja, 5 g ; extrait de levure, 5 g ; NaCl, 5 g ; K₂HPO₄, 5 g pour 900 ml d'eau distillée. Glucose, 100 ml d'une solution à 200 g/L ajoutés au moment de l'ensemencement.
Milieu D : Corn steep liqueur, 10 ml ; peptone de soja, 5 g ; NaNO₃, 2 g ; MgSO₄,7H₂O, 0,5 g ; FeSO₄,7H₂O, 0, 02 g ; KCl, 0,5 g pour 900 ml d'eau distillée. Tampon phosphate (K₂HPO₄, 2 g ; KH₂PO₄, 1 g dans 40 ml d'eau distillée) et glucose, 30 g dans 60 ml d'eau distillée, ajoutés au moment de l'ensemencement.
Milieu E : Milieu C ajusté à pH 7 avec HCl concentré, avant stérilisation.

### EXEMPLE 1 : Etude de différents micro-organismes (Screening)

Pour le screening, les erlens de 250 ml contenant 100 ml de milieu de culture sont ensemencés par une suspension de spores fraîchement récoltés sur milieu solide et la croissance est effectuée dans un incubateur rotatif à 27°C et 200 RPM pendant 66 heures.

La Terfénadine est ajoutée en solution dans l'éthanol (10 ml/litre) à une concentration finale de 0,2 g/l directement à la biomasse dans son milieu de culture.

L'incubation est poursuivie dans les mêmes conditions que la culture pendant 7 jours.

### a) Méthodes analytiques

Des prélèvements réguliers (1 ml par jour) du surnageant sont effectués en cours d'incubation, centrifugés, micro-filtres et injectés en HPLC sur une colonne de nucléosil C18 (250 x 4 mm ; débit : 1 ml/min; détection à 220 nm et à 60°C ; le solvant est un gradient d'acétonitrile-eau-acide trifluoroacétique (TFA) constitué à partir du mélange CH₃CN-eau-TFA (100:900:1) (solvant A) et CH₃CN-eau-TFA (900:100:1) (solvant B).

Dans ces conditions,
la terfénadine (II) a un temps de rétention de 14,56 min., la terfénadine-P (IIIa), un temps de rétention de 11,8 min., le triol (IIIc), un temps de rétention de 9,61 min., la fexofénadine, un temps de rétention de 9,45 min. et le triol-P (IIIb), un temps de rétention de 7,43 min.

### b) Résultats du screening

La réalisation d'un screening dans les conditions de culture décrites plus haut a donné, entra autres souches testées, les résultats reportés dans le tableau ci-dessous et a permis de trouver 2 souches, *Absidia corymbifera* LCP 63-1800 et *Streptomyces platensis* NRRL 2364, capables de produire en erlen la fexofénadine avec un bon rendement, mais en présence d'une quantité non négligeable de triol phosphate et de triol.

**Tableau 1 :**

| Métabolisation de la terfénadine par divers microorganismes, 0,2 g/l, 96 h, 27°C. | | | | | |
|---|---|---|---|---|---|
| | Terfénadine (a) | Triol-P | Fexofénadine | Triol | Terfénadine-P |
| *Absidia blakesleeana* ATCC 6811 | + | - | - | - | - |
| *Absidia blakesleeana* ATCC 42838 | - | ± | - | - | - |
| *Absidia blakesleeana* ATCC 22617 | - | - | - | - | - |
| *Absidia blakesleeana* ATCC 10148b | - | ± | - | - | - |
| *Absidia blakesleeana* ATCC 22739 | - | ± | - | - | - |
| *Absidia corymbifera* LCP 63.1800 | - | ± | +++ | + | - |
| *Absidia corymbifera* LCP 64.1942 | - | - | ± | - | - |
| *Absidia corymbifera* LCP 86.3480 | - | ± | ± | - | - |
| *Actinomucor elegans* MMP 2092 | ± | + | ± | - | - |
| *Aspergillus ochraceus* ATCC 1008 | - | - | ± | - | - |
| *Cunninghamella blakesleeana* ATCC 8688a | ± | ± | - | ± | ± |
| *Cunninghamella echinulata* LCP 73.2203 | ± | - | - | ± | - |
| *Cunninghamella echiniulata* NRRL 3655 | ± | ± | - | ± | ± |
| *Cunninghamella elegans* ATCC 9245 | ± | - | - | - | + |
| *Streptomyces platensis* NRRL 2364 | - | - | + | +++ | - |

| | | | | | |
|---|---|---|---|---|---|
| (a) : Seuls les surnageants de culture ont été testés, ce qui explique l'absence de terfénadine adsorbée sur le mycélium. ± : 5-10 %(HPLC) + : 10-20 % (HPLC) +++ : 70-80 % (HPLC) | | | | | |

### c) Purification et caractérisation complète de la fexofénadine formée par Absidia corymbifera LCP 63.1800

A partir d'un erlen d'Absidia corymbifera (100 ml - 20 mg de terfénadine), après filtration du mycélium et lavage à l'eau, le surnageant est saturé au NaCl puis extrait 3 fois à l'acétate d'éthyle ; la phase organique est séchée sur MgSO₄ puis évaporée sous pression réduite pour conduire à 27 mg d'un résidu solide blanchâtre qui est purifié par chromatographie sur colonne de gel de silice (230-400 mesh) à l'aide du solvant : CH₂Cl₂-MeOH-Ni₄OH (82,5 : 15 : 2,5). On récupère 12,4 mg de produit pur identique à la fexofénadine authentique. F = 190-195°C.

### d) Etude structurale de la fexofénadine obtenue à l'aide d'Absidia corymbifera LCP 63-1800

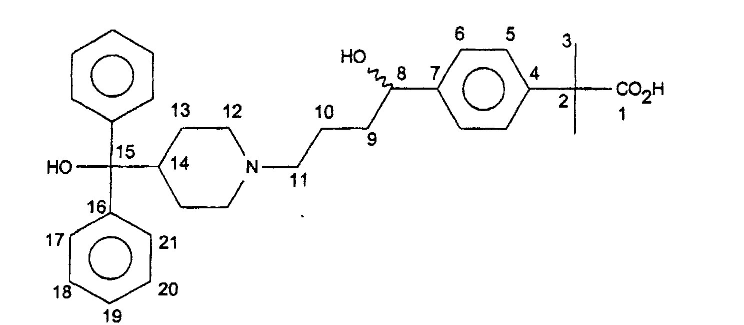

RMN ¹H (CD₃OD, 250,13 MHz) Δ, ppm : 1,49 (6H, s, 2 CH₃) , 1,55-1,82 (8H, m, H-9, H-10 et H-13), 2,73-2,85 (4H, m, H-12), 3,30-3,32 (2H, m, H-11), 4,60 (1H, dd, J=5, 57 et 5, 97 Hz, H-8), 7,17 (2H, d, J=7,17 Hz, H-5), 7,29 (6H, dd, J=7,17 et 7,97 Hz, H-18, H-19 et H-20), 7,39 (2H, d, J=8,37 Hz, H-6), 7,53 (4H, d, J=7,57 Hz, H-17 et H-21).
RMN ¹³C (CD₃OD, 62,9 MHz) Δ, ppm : 23,7 (CH₂, C-10), 27,1 (2 CH₂, C-13), 30,2 (2 CH₃, C-3), 39,0 (CH₂, C-9), 44,9 (CH, C-14), 50,9 (C quat., C-2), 55,4 (2 CH₂, C-12), 59,5 (CH₂, C-11), 75,9 (CH, C-8), 81,7 (C quat., C-15), 23,7 (CH₂, C-10), 128,6 ; 123,9 ; 129,4 et 131,0 (CH, C-5, C-6, C-17, C-18, C-19, C-20 et C-21), 145,2 ; 149,2 et 150,3 (C quat., C-4, C-7 et C-16), 186,3 (C quat., C-1).
MS (electrospray, ions négatifs), m/z : 500 (M-H⁺), 456 (M-H⁺-CO₂) , 378 (456-C₆H₆).

### EXEMPLE 2 : Préparation microbiologique de la Fexofénadine

On ensemence 10 erlens de 250 ml contenant 100 ml de milieu D par *A. corymbifera* LCP 63-1800. On ajoute 50 mg de Terfénadine dans 1 ml d'éthanol dans chaque erlen. A J+7, on filtre les 10 erlens sur gaze (le surnageant a un pH égal à 8,0), et on sature au chlorure de sodium pendant 2 heures (pH = 5-6). On extrait 3 fois à l'acétate d'éthyle et sèche sur sulfate de magnésium. Après évaporation sous pression réduite, on obtient 409 mg de produit brut attendu (rendement = 77 %, produit pur à 90 % environ).
Ce produit est purifié sur colonne de gel de silice (230-400 mesh, 40 g de silice, diamètre = 3 cm) en éluant avec le mélange chlorure de méthylène/méthanol/hydroxyde d'ammonium (82,5-15-2,5). On récupère 312 mg de fexofénadine pure (61,4 %).

### EXEMPLE 3 : Préparation de la fexofénadine en fermenteur (milieu A)

On effectue la culture d'*Absidia corymbifera* dans le milieu A (pH 6,5), mais en fermenteur Biolafitte, dans un volume de 5 litres inoculé avec 2.10⁶ spores/litre, à 27°C, avec un débit d'air de 5 litres/min environ et une agitation (turbine hélicoïdale) de 275 tours/min. La biomasse obtenue après 66 heures de culture et qui se présente sous forme de pellets homogènes de 1 mm environ de diamètre a été utilisée pour la bioconversion directement dans le fermenteur de culture. 1,25 g de Terfénadine dans 50 ml d'éthanol sont ajoutés et l'incubation est poursuivie dans les mêmes conditions pendant 7 jours. A ce stade, le pH est de 8,5 et le milieu d'incubation contient 35 % de fexofénadine et 65 % de triolphosphate. La biomasse est séparée du milieu liquide par filtration. Le filtrat est saturé en NaCl, son pH est ajusté à 6,0 avec HCl dilué et il est extrait 3 fois à l'acétate d'éthyle. Après purification comme décrit dans l'exemple 2, on récupère 350 mg (27 %) de fexofénadine pure. On peut récupérer le triolphosphate (IIIb) dans la phase aqueuse par adsorption sur une colonne de XAD-2 et élution avec le méthanol.

### EXEMPLE 3 bis : Préparation de la fexofénadine en fermenteur (milieu D)

On effectue la culture d'*Absidia corymbifera* dans le milieu D (pH 6,5), en fermenteur Biolafitte, dans les mêmes conditions que dans l'exemple 3. La biomasse obtenue après 66 heures de culture est utilisée pour la bioconversion directement dans le fermenteur de culture. 1 g de Terfénadine dans 25 ml d'éthanol sont ajoutés et l'incubation est poursuivie dans les mêmes conditions pendant 4 jours. A ce stade, le pH est de 8,0 et le milieu d'incubation contient 70 % de fexofénadine et 30 % de triolphosphate qui sont isolés et purifiés comme décrit dans l'exemple 3.

### EXEMPLE 4 : Méthode avec ajustements successifs du pH pour la préparation de la fexofénadine en fermenteur

On effectue la culture d'*Absidia corymbifera* dans le milieu D (pH 6,5), dans les conditions décrites dans l'exemple 3. 2,5 g de Terfénadine dans 50 ml d'éthanol sont ajoutés et l'incubation est poursuivie dans les mêmes conditions pendant 4 jours. A ce stade, le pH est de 8,0 et le milieu d'incubation contient 42 % de fexofénadine, 13 % de triol (IIIc) et 25 % de triolphosphate (IIIb). Le pH est ajusté à 3,5 avec HCl concentré et on continue l'incubation dans les mêmes conditions en laissant le pH du milieu évoluer librement jusqu'à 6 (3 jours). A ce stade, le triol-phosphate (IIIb) a presque totalement disparu au bénéfice du triol. On réajuste le pH à 8,0 et on poursuit l'incubation dans les mêmes conditions pendant 2 jours supplémentaires. Le milieu contient alors 85 % de fexofénadine et 13 % de triol phosphate (IIIb).

### EXEMPLE 5 : Méthode optimisée pour la préparation de la fexofénadine en fermenteur

On effectue la culture d'*Absidia corymbifera* dans le milieu D (pH 6,5), dans les conditions décrites dans l'exemple 3. 2,5 g de Terfénadine dans 50 ml d'éthanol sont ajoutés et l'incubation est poursuivie dans les mêmes conditions pendant 4 jours. A ce stade, le pH est de 8,0 et le milieu d'incubation contient 35 % de fexofénadine, 11,5 % de triol (IIIc) et 34 % de triolphosphate (IIIb). Le pH est ajusté et maintenu à 6,0-6,5 avec HCl dilué pendant qu'on continue l'incubation dans les mêmes conditions (3 jours). Le milieu contient alors 89,5 % de fexofénadine et 9,5 % de triol phosphate.

### EXEMPLE 6 :

On ensemence un erlen de 250 ml contenant 100 ml de milieu E par *Streptomyces platensis* NRRL 2364 et on cultive comme décrit dans l'exemple 1. Une moitié du milieu de culture et de la biomasse, dont le pH est de 5,0, est additionnée de 10 mg de terfénadine dissoute dans 0,5 ml d'éthanol et l'incubation est poursuivie pendant plusieurs jours dans les mêmes conditions. Après 3 jours, le seul métabolite observé est le triol (IIIc) (60 %) ; après 7 jours le rendement en triol atteint 67 %.

### EXEMPLE 7 :

L'autre moitié du milieu de culture et de la biomasse de l'exemple 6, est additionnée de terfénadine (10 mg dans 0,5 ml d'éthanol) et incubé dans les mêmes conditions que dans l'exemple 6, pendant 3 jours, puis porté à pH 8,0 par addition de NaOH 7,5 M. L'incubation est poursuivie pendant 4 jours supplémentaires et on obtient 16 % de triol-phosphate (IIIb), 37 % de fexofénadine (I) et 26 % de triol (IIIc).

### Exemple 8 : Préparation de la féxofénadine en fermenteur

### Préculture

La préculture est réalisée en milieu minimum (la source de carbone est le glucose) en flacons d'Erlenmeyer de 500 ml contenant 100 ml de milieu stérile. Les erlens sont ensemencés par 450 µl d'une suspension glycérolée de *Streptomyces platensis* NRRL 2364 (stock congelé à -80°C) et incubés en shaker orbital (2,5 cm d'excentration) à 34°C, 250 RPM pendant 75 heures.

### Fermentation / Bioconversion

La fermentation est réalisée en fermenteurs de volume total 500 ml contenant 300 ml de milieu minimum à 125 g/l de glucose. Le fermenteur est inoculé par 7,5 ml de la préculture décrite ci-dessus. Les conditions de culture sont les suivantes :
· température régulée à 34°C
· agitation constante (pas de régulation de pO2)
· aération constante à 54 l/h (3vvm)
· pH régulé à 6.0 par ajout de KOH à 20%

A 48 heures de culture, on ajoute 75 mg de terfénadine solubilisée dans 3 ml d'éthanol. La culture est poursuivie jusqu'à 212 heures d'âge.

### Résultats

Pour cet essai, on dose la terfénadine et ses dérivés par HPLC reverse après dilution des bouillons au 1/10 dans l'éthanol. On obtient à 212 heures 71% d'avancement de réaction (féxofénadine formée) et un bilan estimé à 82%.

**Tableau 2 :**

| | |
|---|---|
| Vitesse initiale d'oxydation de la Terfénadine en fonction du pH (Biomasse de 66 h, lavée et remise en suspension dans un tampon phosphate 0,05 M en présence de terfénadine 1061 µmol/litre). La mesure effectuée par HPLC correspond à la somme (triol + triol-phosphate + fexofénadine) | |

| pH | Terfénadine oxydée (total) µmol/l/h |
|---|---|
| 6,3 | 1,60 |
| 6,5 | 2,45 |
| 7,0 | 0,60 |
| 7,5 | 0,59 |
| 8,0 | 0,76 |

**Tableau 3 :**

| | |
|---|---|
| Vitesse initiale (après 2 heures d'incubation) de phosphorylation du triol en fonction du pH (Biomasse de 66 h, lavée et remise en suspension dans un tampon phosphate 0,05 M en présence de triol 186 µmol/litre). On mesure par HPLC le triol-phosphate formé dans le surnageant d'incubation (aucune formation de fexofénadine n'est observée dans les 2 premières heures). | |

| pH | Triol-phosphate µmol/l/h |
|---|---|
| 6,3 | 53,5 |
| 6,5 | 57,0 |
| 7,0 | 41,5 |
| 7,5 | 27,0 |
| 8,0 | 24,0 |

**Tableau 4 :**

| | | | | | |
|---|---|---|---|---|---|
| Vitesse initiale d'hydrolyse du triol-phosphate en fonction du pH (surnageant d'incubation de 120 heures contenant 272 µmol/litre de triol-phosphate, ajusté et maintenu aux différents pHs par HCl concentré et incubé à 27°C. L'hydrolyse est mesurée en HPLC à la fois par la disparition de triol-phosphate et l'apparition de triol. La vitesse initiale est calculée sur la moyenne des prélèvements effectués pendant les 7,5 premières heures d'hydrolyse. | | | | | |

| | Après 4 heures d'incubation | | Après 24 heures d'incubation | | Vitesse initiale d'hydrolyse du triol-phosphate µmol/l/h |
|---|---|---|---|---|---|
| | triol-phosphate résiduel µmol/l (% hydrolyse) | triol apparu µmol/l (% hydrolyse) | trial-phosphate résiduel µmol/l (% hydrolyse) | triol apparu µmol/l (% hydrolyse) | |
| 4,0 | 258 (5 %) | 12 (4,4 %) | 223 (18 %) | 35 (13 %) | 3,13 |
| 5,0 | 234 (14 %) | 40 (14,7 %) | 148 (45,6%) | 120 (44,1%) | 8,71 |
| 6,0 | 196 (27,9%) | 68 (25 %) | 50 (81,7%) | 216 (79,4%) | 17,49 |
| 6,5 | 224 (17,6%) | 30 (11 %) | 140 (48,5%) | 131 (48,2%) | 8,71 |
| 7,0 | 237 (12, 9%) | 30 (11 %) | 167 (38,6%) | 109 (40 %) | 7,12 |
| 7,5 | 240 (12 %) | 31 (11,4 %) | 181 (33 %) | 77 (28,3 %) | 5,48 |
| 8,0 | 244 (10 %) | 19 (7 %) | 197 (28 %) | 69 (25,4 %) | 4,37 |

**Tableau 5 :**

| | | | |
|---|---|---|---|
| oxydation du triol mesurée entre 6 h et 24 h d'incubation en fonction du pH (Biomasse de 66 h, lavée et remise en suspension dans un tampon phosphate 0,05 M en présence de triol 186 µmol/litre). Dans cet intervalle de temps où la fexofénadine est formée, la concentration en triol est stationnaire. Les deux produits sont mesurés par HPLC. | | | |

| pH | Triol aux temps 6-24 h µmol/litre | Triol-phosphate aux temps 6-24 h µmol/litre | Fexofénadine apparue entre 6 et 24 h µmol/litre (%)* |
|---|---|---|---|
| 6,3 | 110-130 | 65-27 | 0 (0) |
| 6,5 | 93-96 | 72-31 | 15 (15,6) |
| 7,0 | 46-58 | 110-64 | 25 (45,1) |
| 7,5 | 21-43 | 124-61 | 30 (69,7) |
| 8,0 0 | 20-49 | 128-41 | 30 (61, 2) |

| | | | |
|---|---|---|---|
| * % calculé = (fexofénadine formée/triol résiduel) x 100 | | | |

Figure 1 : Récapitulation de l'influence du pH sur les quatre réactions unitaires du métabolisme de la terfénadine par *A. blakesleeana*. L'échelle des ordonnées a été ramenée par multiplication pour chaque courbe à des valeurs du même ordre.
Figure 2 : Bioconversion de la terfénadine (0,2 g/l) en milieu D, sans contrôle de pH.
Figure 3 : Bioconversion de la terfénadine (0,5 g/l) en erlen de 100 ml en milieu D avec ajustement du pH de l'incubation à 3,5 (96 h) et 8,0 (168 h).
Figure 4 : Bioconversion de la terfénadine (0,5 g/l 27°C, milieu D) en erlen par *A. blakesleeana* avec modification et maintien du pH de l'incubation à 6,0 après 96 heures.

## Revendications

1. Procédé de préparation des composés de formule (I) : 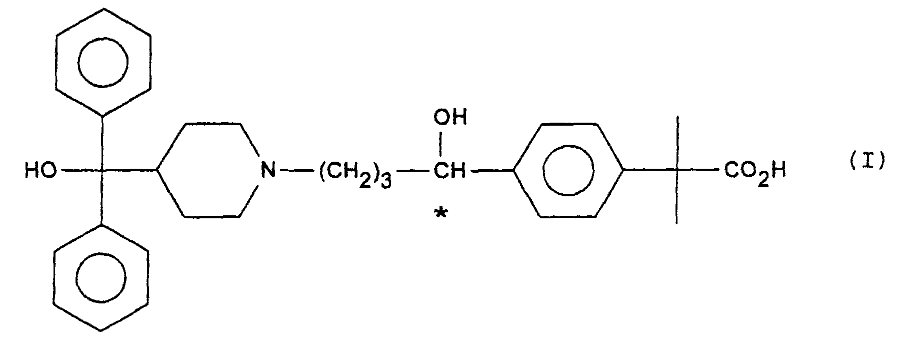 **caractérisé en ce qu'**on effectue une bioconversion du ou des composés de formule (II) : 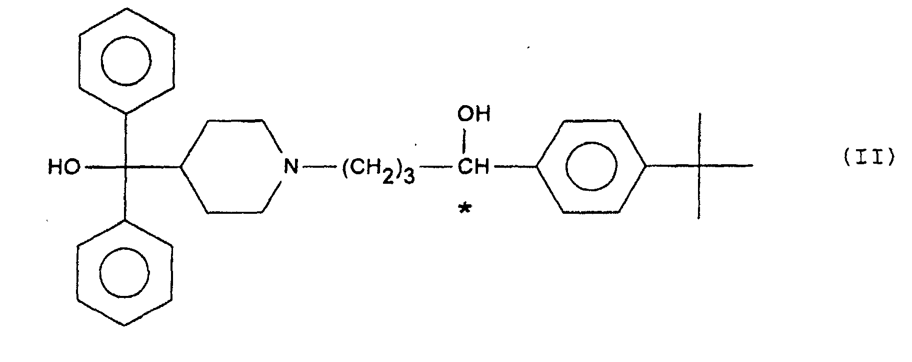 avec soit une culture d'un microorganisme de genre *Absidia corymbifera* soit une culture d'un microorganisme de genre *Streptomyces*, à un pH compris entre 5,0 et 8,0, afin d'obtenir le ou les composés de formule (I) attendus, qui le cas échéant sont isolés, purifiés et/ou salifiés, les composés de formules (I) ou (II) pouvant être sous les deux formes énantiomères possibles, isolés ou en mélanges.

2. Procédé selon la revendication 1 dans lequel l'*Absidia corymbifera* est *Absidia corymbifera* LCP 63-1800.

3. Procédé selon la revendication 1 dans lequel le *Streptomyces* est *Streptomyces platensis* NRRL 2364.

4. Procédé selon la revendication 1, 2 ou 3 dans lequel on effectue une bioconversion de la Terfénadine, correspondant à un mélange racémique des énantiomères de formule (II), afin d'obtenir la Fexofénadine, correspondant à un mélange racémique des énantiomères de formule (I).

5. Procédé selon l'une quelconque des revendications 1 à 4 dans lequel les conditions de biotransformation sont les suivantes :
- concentration en Terfénadine ajoutée d'environ 0,5 à 10 g/l
- pH évoluant entre 5,0 et 8,0
- température comprise entre 26°C et 28°C
- aération par introduction d'un débit d'air d'environ 1 l/minute et par litre de bouillon de culture.

6. Procédé selon l'une quelconque des revendications 1 à 5 dans lequel le pH initialement à environ 6,5,
- évolue naturellement à 8,0-8,5, ce qui conduit à un mélange de triol-phosphate (IIIb) : 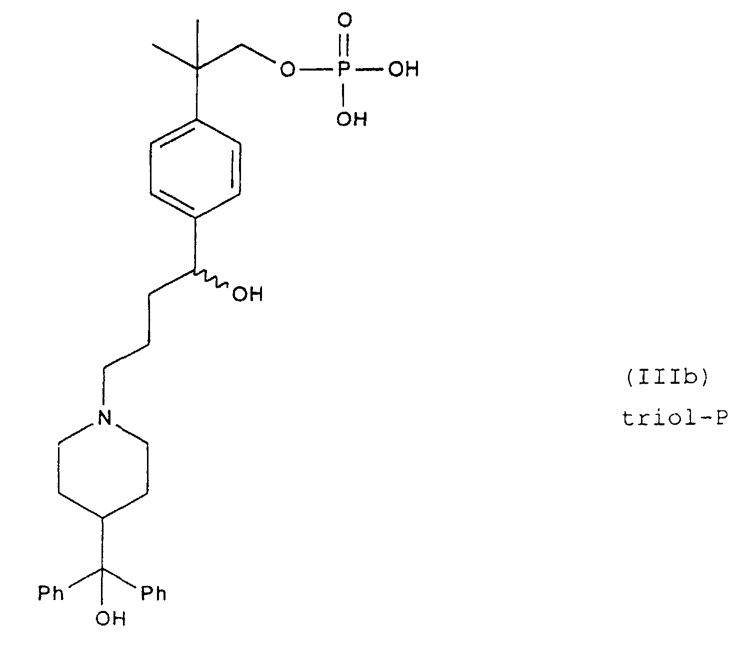 et de composé de formule (I),
- est ensuite abaissé à une valeur comprise entre 3,5 et 4 afin de favoriser la transformation du composé intermédiaire de formule (IIIb) en composé intermédiaire de formule (IIIc) : 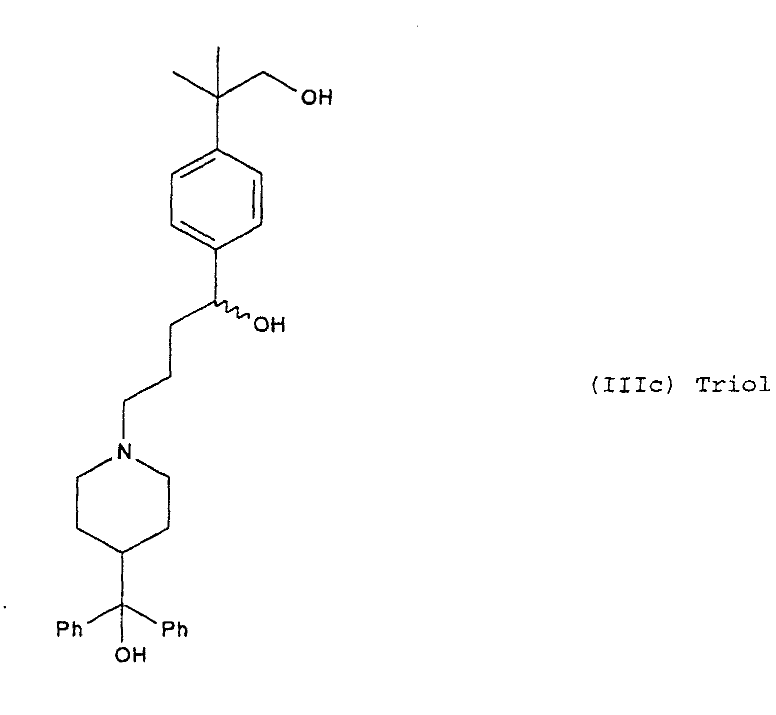
- puis évolue naturellement à environ 6,0 jusqu'à disparition complète du composé (IIIb),
- et enfin est réajusté et maintenu à environ 8,0 jusqu'à transformation du composé (IIIc) en Fexofénadine.

7. Procédé selon l'une quelconque des revendications 1 à 5 dans lequel le pH initialement à environ 6,5
- évolue naturellement à 8,0-8,5,
- puis est abaissé et maintenu à une valeur comprise entre 6,3 et 6,8.

8. Procédé selon l'une quelconque des revendications précédentes **caractérisé en ce que**, au cours de l'étape de purification, l'extraction du produit de formule (I) tel que défini à la revendication 1, est effectuée dans l'acétate d'éthyle.

9. A titre de composé intermédiaire le composé de formule (IIIc) telle que définie à la revendication 6.

## Patentansprüche

1. Verfahren zur Herstellung der Verbindungen der Formel (I): 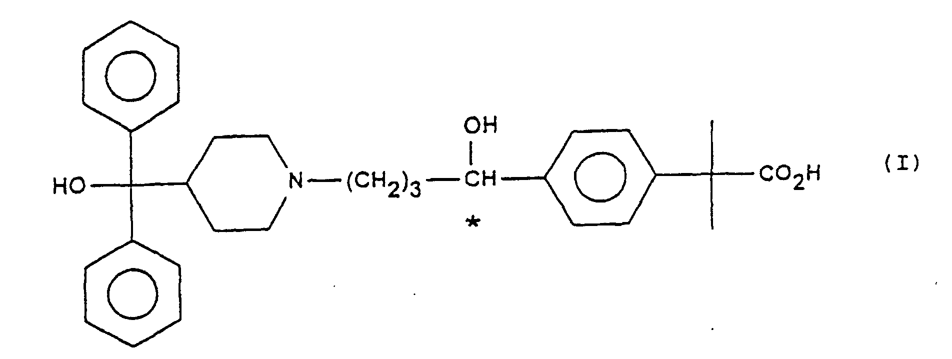 **dadurch gekennzeichnet, daß** man eine Biokonversion der Verbindung(en) der Formel (II): 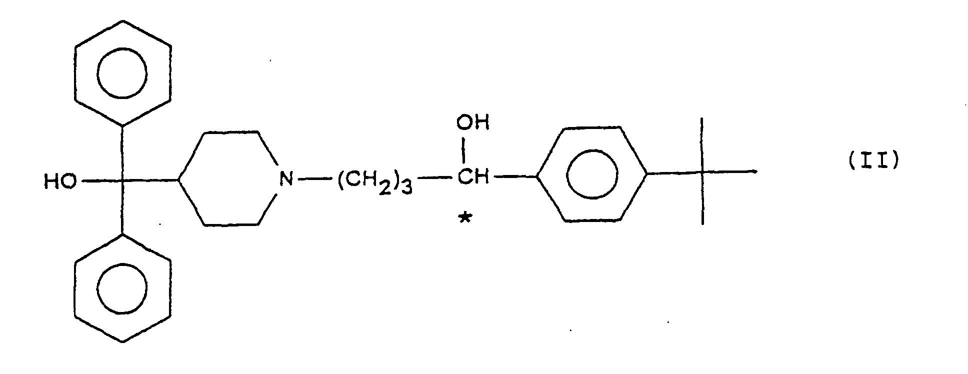 entweder mit einer Kultur eines Mikroorganismus der Gattung Absidia corymbifera oder einer Kultur eines Mikroorganismus der Gattung Streptomyces bei einem pH von 5,0 bis 8,0 durchführt, um die erwartete(n) Verbindung(en) der Formel (I) zu erhalten, die gegebenenfalls isoliert, gereinigt und/oder in ein Salz übergeführt wird/werden, wobei die Verbindungen der Formel (I) oder (II) in den zwei möglichen enantiomeren Formen, isoliert oder in Gemischen, vorliegen können.

2. Verfahren nach Anspruch 1, wobei Absidia corymbifera LCP 63-1800 ist.

3. Verfahren nach Anspruch 1, wobei Streptomyces Streptomyces platensis NRRL 2364 ist.

4. Verfahren nach Anspruch 1, 2 oder 3, wobei eine Biokonversion von Terfenadin, das einem racemischen Gemisch der Enantiomeren der Formel (II) entspricht, unter Erhalt von Fexofenadin, das einem racemischen Gemisch der Enantiomeren der Formel (I) entspricht, durchgeführt wird.

5. Verfahren nach einem der Ansprüche 1 bis 4, wobei die Biotransformationsbedingungen die folgenden sind:
- Konzentration des zugesetzten Terfenadin etwa 0,5 bis 10 g/l;
- pH, der sich zwischen 5,0 und 8,0 bewegt,
- Temperatur zwischen 26°C und 28°C,
- Belüftung durch Einleitung eines Luftstroms mit einem Durchfluß von 1 1/Minute und Liter Kulturbrühe.

6. Verfahren nach einem der Ansprüche 1 bis 5, wobei der pH von anfangs 6,5 sich natürlicherweise auf 8,0 bis 8,5 entwickelt, was zu einem Gemisch aus Triolphosphat (IIIb): 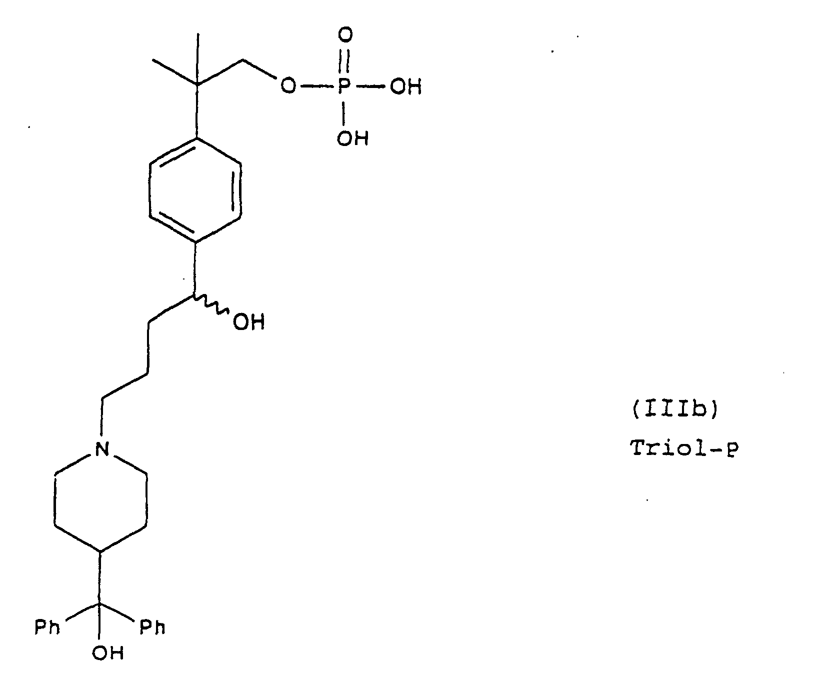 und der Verbindung der Formel (I) führt,
- dann auf einen Wert zwischen 3,5 und 4 gesenkt wird, um die Transformation der-Zwischenverbindung der Formel (IIIb) zu Zwischenverbindung der Formel (IIIc) zu begünstigen: 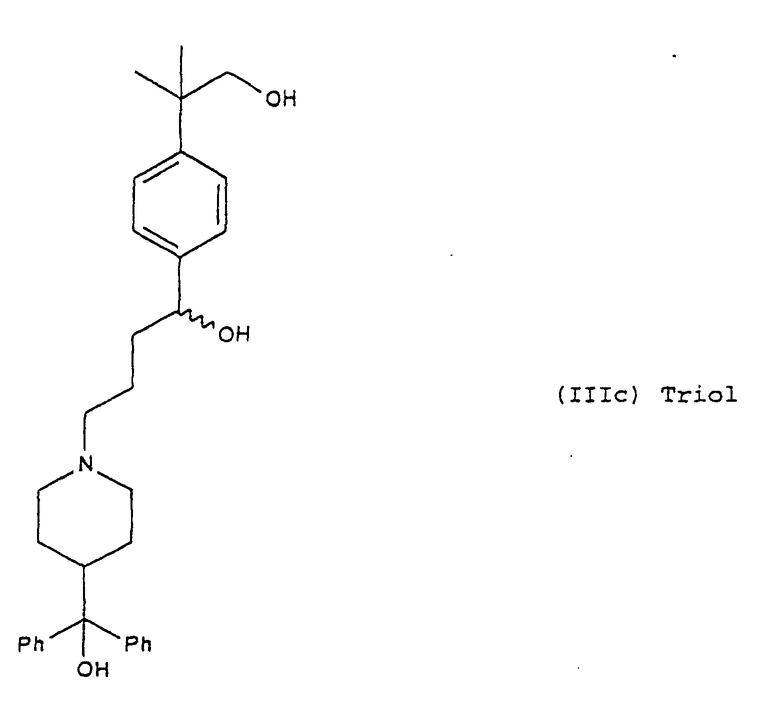
- der pH sich dann natürlicherweise auf etwa 6,0 bis zum vollständigen Verschwinden der Verbindung (IIIb) entwickelt,
- und schließlich wieder auf 8,0 eingestellt und bis zur Transformation der Verbindung (IIIc) in Fexofenadin dort gehalten wird.

7. Verfahren nach einem der Ansprüche 1 bis 5, wobei der pH anfangs etwa 6,5 ist,
- sich natürlicherweise auf 8,0 bis 8,5 entwickelt,
- dann auf einen Wert zwischen 6,3 und 6,8 erniedrigt wird und bei diesem Wert gehalten wird.

8. Verfahren nach einem der vorangehenden Ansprüche, **dadurch gekennzeichnet, daß** im Verlauf der Reinigungsstufe die Extraktion des Produktes der Formel (I), wie es in Anspruch 1 definiert ist, in Ethylacetat durchgeführt wird.

9. Verbindung der Formel (IIIc), wie sie in Anspruch 6 definiert ist, als Zwischenverbindung.

## Claims

1. Process for the preparation of the compounds of formula (I) 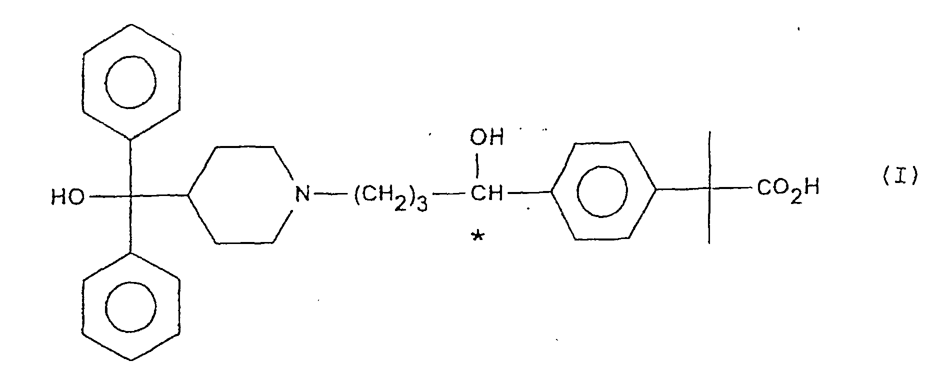 **characterized in that** a bioconversion of the compound or compounds of formula (II) is carried out: 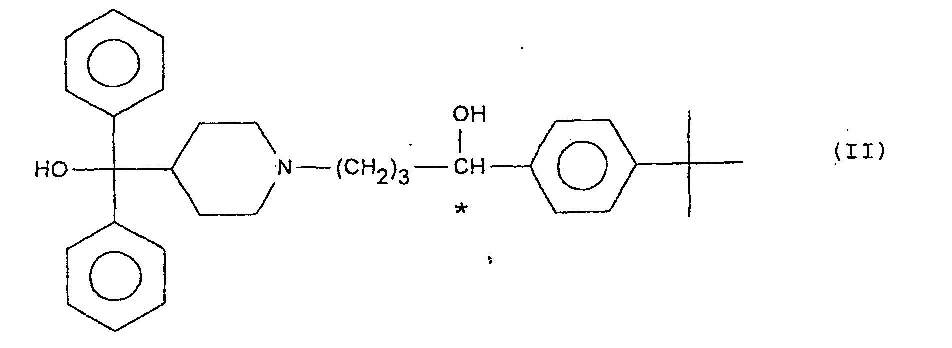 with either a microorganism culture of the genus *Absidia corymbifera* or a microorganism culture of the genus *Streptomyces,* at a pH comprised between 5.0 and 8.0, in order to obtain the expected compound or compounds of formula (I), which if appropriate are isolated, purified and/or salified, the compounds of formulae (I) or (II) being able to be in the two possible enantiomeric forms, isolated or in mixtures.

2. Process according to claim 1 in which the *Absidia corymbifera* is *Absidia corymbifera* LCP 63-1800.

3. Process according to claim 1 in which the *Streptomyces* is *Streptomyces platensis* NRRL 2364.

4. Process according to claim 1, 2 or 3 in which a bioconversion of Terfenadine is carried out, corresponding to a racemic mixture of the enantiomers of formula (II), in order to obtain the Fexofenadine, corresponding to a racemic mixture of the enantiomers of formula (I).

5. Process according to any one of the claims 1 to 4 in which the biotransformation conditions are as follows:
- concentration of added Terfenadine of approximately 0.5 to 10 g/l
- pH evolving to between 5.0 and 8.0
- temperature comprised between 26°C and 28°C
- aeration by introduction of an airflow of approximately 1 l/minute and per litre of broth culture medium.

6. Process according to any one of the claims 1 to 5 in which the initial pH is approximately 6.5,
- evolves naturally to 8.0-8.5, which leads to a mixture of triol-phosphate (IIIb): 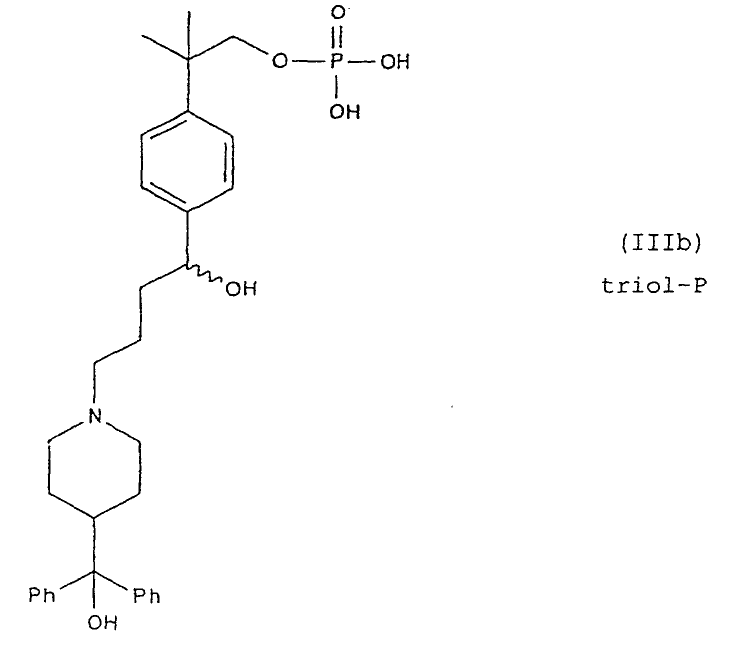 and compound of formula (I),
- is then reduced to a value comprised between 3.5 and 4 in order to encourage the transformation of the intermediate compound of formula (IIIb) to the intermediate compound of formula (IIIc): 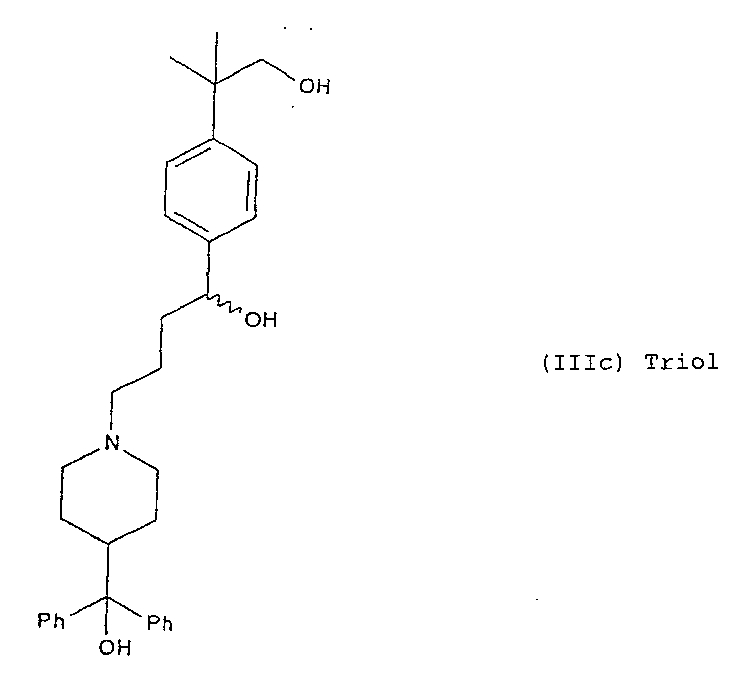
- then evolves naturally to approximately 6.0 until compound (IIIb) has completely dispersed,
- and finally is readjusted and maintained at approximately 8.0 until transformation of compound (IIIc) to Fexofenadine.

7. Process according to any one of the claims 1 to 5 in which the initial pH is approximately 6.5
- evolves naturally to 8.0-8.5,
- then is reduced and maintained at a value comprised between 6.3 and 6.8.

8. Process according to any one of the previous claims **characterized in that**, during the purification stage, extraction of the product of formula (I) as defined in claim 1 is carried out in ethyl acetate.

9. As an intermediate compound, the compound of formula (IIIc) as defined in claim 6.
